(19) Europäisches Patentamt
European Patent Office
Office européen des brevets

(11) Veröffentlichungsnummer: **0 099 021**
**B1**

(12) # EUROPÄISCHE PATENTSCHRIFT

(45) Veröffentlichungstag der Patentschrift: **18.12.85**

(51) Int. Cl.⁴: **C 07 D 327/04**, C 07 D 327/06

(21) Anmeldenummer: **83106261.7**

(22) Anmeldetag: **28.06.83**

(54) **Verfahren zur Herstellung von Alkansultonen.**

(30) Priorität: **10.07.82 DE 3225887**

(43) Veröffentlichungstag der Anmeldung: **25.01.84 Patentblatt 84/4**

(45) Bekanntmachung des Hinweises auf die Patenterteilung: **18.12.85 Patentblatt 85/51**

(84) Benannte Vertragsstaaten: **BE CH DE FR GB LI**

(56) Entgegenhaltungen:
**DE - C - 887 341**
**DE - C - 902 615**

(73) Patentinhaber: **AGFA-GEVAERT Aktiengesellschaft, D-5090 Leverkusen 1 (DE)**

(72) Erfinder: **Kampfer, Helmut, Dr., Roggendorfstrasse 63, D-5000 Köln 80 (DE)**
Erfinder: **Himmelmann, Wolfgang, Dr., Im Ziegelfeld 7, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 099 021 B1

**Beschreibung**

Die Erfindung betrifft ein Verfahren zur Herstellung von Alkansultonen, insbesondere von 1,3-Propansultonen und 1,4-Butansultonen.

Sultone, deren Namen sich von Hydroxysulfonsäurelacton ableitet, sind seit langem bekannt. In der Literatur wurden Vertreter in der aromatischen, der hydroaromatischen und aliphatischen Reihe beschrieben (vgl. die Übersicht von J. Willems, „Industrie Chimique Belge", T. XIX, S. 905, 1954). Insbesondere die aliphatischen Sultone (Alkansultone) haben wegen ihrer Fähigkeit zu Alkylierungsreaktionen verbreitetes technisches Interesse gefunden. Bei diesen Reaktionen werden Sulfoalkylreste eingeführt, die zu einer starken Erhöhung der Hydrophilie oder sogar zu einer leichten Wasserlöslichkeit des Reaktanden beitragen. Aus diesem Grunde werden Sulfoalkylverbindungen zur Herstellung von Detergentien in der Waschmittelindustrie, in der Galvanotechnik und auch zur Herstellung von sulfoalkylierten Farbstoffen verwendet. Zu den sulfoalkylierten Farbstoffen von erheblichem technischen Interesse gehören auch heterocyclische Farbstoffe der Polymethin- oder Cyanin-Klasse, die als Spektralsensibilisatoren in der Fotografie gebraucht werden. Zu deren Herstellung müssen N-haltige heterocyclische Basen sulfoalkyliert werden, die gegen Mineralsäuren und Hydrolyse teilweise sehr empfindlich sind. Bei diesen Sulfoalkyl-substituierten Polymethinfarbstoffen ist der Einsatz von Sultonen als Sulfoalkylierungsmitteln Voraussetzung für gute Zugänglichkeit.

An aliphatischen Sultonen sind insbesondere die fünfgliedrigen Propansultone, insbesondere 1,3-Propansulton, und die sechsgliedrigen Butansultone, insbesondere 1,4-Butansulton, von Bedeutung. Zu ihrer Herstellung sind sehr unterschiedliche Verfahren beschrieben worden. So können Propan- und Butansulton durch Sulfochlorierung von 1-Chlorpropan bzw. 1-Chlorbutan mit Schwefeldioxid und Chlor unter Belichtung über die Chlorpropan- bzw. Chlorbutansulfochloride in die entsprechenden Chlorpropan- bzw. Chlorbutansulfonsäuren übergeführt werden, die thermisch unter HCl-Abspaltung in die Sultone übergehen [vgl. J.H. Helberger *et al.*, „Liebigs Ann. Chem.", *562* (1949) 23]. Ein weiteres Verfahren basiert auf der Addition von Bisulfiten an ungesättigte Alkohole wie Allylalkohol und thermische Wasserabspaltung aus der entstandenen Hydroxyalkansulfonsäure [vgl. J.H. Helberger, „Liebigs Ann. Chem.", *588* (1954) 71 und J. Willems, „Bull. Soc. Chim. Belg.", *64* (1955) 747] unter Bildung von Sulton.

Auch die Addition von Bisulfiten an ungesättigte Aldehyde mit nachfolgender Hydrierung zu den Hydroxysulfonsäuren als Ausgangsverbindungen zur Sultonherstellung ist beschrieben [vgl. C. Smith *et al.*, „J. Amer. Chem. Soc.", *75* (1953), 748]. Ein weiteres Verfahren betrifft die Synthese des 1,4-Butansultons. Es geht aus von dem durch Spaltung von Tetrahydrofuran gut zugänglichen 4,4'-Dichlordibutylether, der sich mit Sulfit in die entsprechende Dibutylether-4,4'-disulfonsäure und daraus thermisch in 1,4-Butansulton überführen lässt [vgl. J.H. Helberger und H. Lantermann, „Liebigs Ann. Chem.", *586* (1954), 158 oder „Org. Synthesis Coll.", vol. IV (1963), 529].

Auch die Synthese des 2,4-Butansultons aus der entsprechenden Methoxybutansulfonsäure durch thermischen Ringschluss ist bereits bekannt [vgl. J.H. Helberger *et al.*, „Liebigs Ann. Chem.", 586 (1954), 155]. Doch wird bei der Reaktion Methanol frei, das in vielen Fällen bei Folgereaktionen stört. All diesen bekannten Verfahren haften als wesentliche Nachteile an, dass bei ihrer Anwendung Wasser, Alkohole oder Mineralsäuren frei werden und damit bei Verwendung der Sultone als Sulfoalkylierungsmittel für solvolyse- oder mineralsäureempfindliche Verbindungen vorher destilliert werden müssen. Eine Destillation der Sultone ist jedoch aus Kostengründen und wegen der mit dem z. T. erheblichen cancerogenen Potential der meisten Sultone zusammenhängenden Sicherheitsrisiken möglichst zu vermeiden.

Aus diesen Gründen bestand ein Interesse an einem Verfahren zur Herstellung der Alkansultone, das nicht mit den genannten Nachteilen behaftet ist.

Gegenstand der Erfindung ist ein Verfahren zur Herstellung von Alkansultonen der allgemeinen Formel I:

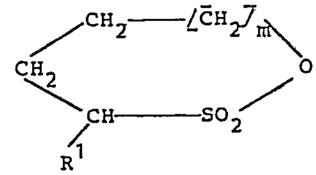

(I)

worin bedeuten

$R^1$ Wasserstoff oder Methyl, und

m 0 oder 1,

gekennzeichnet durch thermische Spaltung von Sulfoalkylarylethern der allgemeinen Formel II bei erhöhter Temperatur:

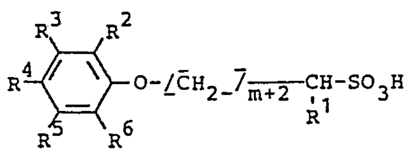

(II)

worin bedeuten

$R^1$ Wasserstoff oder Methyl,

m 0 oder 1, und

$R^2$, $R^3$, $R^4$, $R^5$ und $R^6$ Wasserstoff oder gleiche oder verschiedene Substituenten, und zwar Methyl oder Ethyl, Halogen, z. B. F, Cl, Br oder I, oder Alkoxy, vorzugsweise Methoxy, oder

$R^3$ bedeutet zusammen mit $R^2$ oder zusammen mit $R^4$ einen Rest zur Vervollständigung eines ankondensierten gesättigten oder ungesättigten, 5- oder 6gliedrigen, ggf. Sauerstoff- oder Schwefelatome enthaltenden Ringes.

Die Substituenten $R^2$, $R^3$, $R^4$, $R^5$ und $R^6$ sind im übrigen hinsichtlich Art und Grösse für das erfindungsgemässe Verfahren nur insoweit von Bedeutung, als sie die thermische Spaltung der Sulfoalkylarylether und ggf. die Entfernung der abgespaltenen Phenole nicht beeinträchtigen sollen. Es wird deshalb darauf zu achten sein, dass diese Substituenten möglichst klein sind, so dass die bei der thermischen Spaltung freigesetzten Phenole leicht durch Destillation entfernt werden können.

Die Reaktion erfolgt bei einer Temperatur zwischen 80 und 250° C, vorzugsweise zwischen 140 und 170° C. Zweckmässig wird das bei der Spaltung entstehende Phenol im Vakuum abdestilliert, wobei der Druck so einzustellen ist, dass das Phenol rasch abdestillieren kann und das gebildete Sulton im Reaktionsgefäss verbleibt und dort weiter umgesetzt werden kann. Das gebildete Sulton kann aber auch wie bei den bekannten Verfahren unter vermindertem Druck destilliert werden. Die destillative Trennung von Phenol und Sulton bereitet wegen der erheblichen Siedepunktsdifferenzen keine Schwierigkeiten. Eine Kolonne ist im allgemeinen nicht erforderlich.

Der besondere Vorteil des erfindungsgemässen Verfahrens besteht darin, dass das bei der Reaktion als Nebenprodukt gebildete Phenol im Gegensatz zu den bei den bekannten Verfahren zur Herstellung von Alkansultonen entstehenden Nebenprodukten nur geringe solvolytische Reaktionsfähigkeit besitzt und seine Anwesenheit daher bei den folgenden Umsetzungen in den meisten Fällen nicht stört. Daher kann das Sulton im gleichen Gefäss, in dem es erzeugt wurde, weiterverarbeitet werden; das bedeutet, dass die Sulfoalkylierungen sich in geschlossenen Apparaturen durchführen lassen, ohne dass die Alkansultone transportiert oder umgefüllt werden müssen, so dass eine Kontaminierung der Atmosphäre mit Sicherheit vermieden werden kann.

Dies gilt auch dann, wenn das gebildete Alkansulton in einer geschlossenen Apparatur einer destillativen Reinigung unterzogen und in der Destillationsvorlage weiter umgesetzt wird. Der glatte Reaktionsverlauf der thermischen Spaltung macht die Sulfoalkylarylether zu geeigneten Vorläuferverbindungen für die als Sulfoalkylierungsmittel verwendeten Alkansultone.

Beispiele für geeignete Sulfoalkylarylether als Ausgangsmaterialien für die Herstellung von Alkansultonen sind im folgenden aufgeführt:

(3-Sulfopropyl)phenylether (Ether 1), Fp. 70-72° C;
3-Sulfopropyl-(3-methylphenyl)ether (Ether 2);
(3-Sulfopropyl)-(3-methyl-4-chlorphenyl)ether (Ether 3), Fp. 43-50° C;
(3-Sulfopropyl)-(4-methylphenyl)ether (Ether 4), Fp. 74-75° C;
(3-Sulfopropyl)-(1-naphthyl)ether (Ether 5), Fp. 48-60° C;
(3-Sulfopropyl)-(3-Chlorphenyl)ether (Ether 6;
(3-Sulfopropyl)-(3-nitrophenyl)ether (Ether 7);
(3-Sulfobutyl)-(1-naphthyl)ether (Ether 8), Fp. 86-87° C;
(4-Sulfobutyl)-(3-methylphenyl)ether (Ether 9);
(3-Sulfobutyl)-(3-methylphenyl)ether (Ether 10).

Die erfindungsgemässen Sulfoalkylarylether lassen sich herstellen durch Umsetzung von ω-Halogenalkylarylether mit $Na_2SO_3$ [vgl. Schenck und Kaizerman, „J. Amer. chem. Soc." *75* (1953) 1636, 1641] oder durch Umsetzung von ω-Halogenalkansulfonsäuren mit Natrium- oder Kaliumphenolaten.

Nicht zuletzt können die Sulfoalkylarylether auch durch Umsetzung von Natrium- oder Kaliumphenolaten mit Alkansultonen erhalten werden. Sie stellen somit eine physiologisch und ökologisch unbedenkliche Transportform für die Alkansultone dar.

Das erfindungsgemässe Verfahren wird durch die nachstehenden Beispiele erläutert.

*Beispiel 1:*

*1,3-Propansulton*

a) 254 g K-3-Sulfopropylphenylether (Kalium-3-phenoxypropansulfonat) (oder 238 g Na-3-Sulfopropylphenylether) wurden in 0,6 l Aceton suspendiert, mit 110 g konzentrierter Salzsäure versetzt und 1 h bei Raumtemperatur gerührt. Das ausgefallene KCl (NaCl) wurde abfiltriert und aus dem Filtrat wurde bei Normaldruck das Aceton abdestilliert. Anschliessend wurde die vebleibende 3-Phenoxypropansulfonsäure durch Erhitzen auf 140-160° C bei 7 mb zersetzt, wobei das gebildete Phenol bei 90-120° C Kopftemperatur abdestillierte. Im Rückstand verblieb ein Gemisch von 0,63 mol Propansulton und 0,32 mol 3-Phenoxypropansulfonsäure sowie Reste nicht destillierten Phenols. Das Gemisch konnte ohne weitere Reinigung anstelle von 1,3-Propansulton als Sulfopropylierungsmittel verwendet werden. Werden derartige Reaktionen nach einem der bekannten Verfahren ohne Destillation des Sultons durchgeführt, so führen sie entweder überhaupt nicht oder nur in geringerer Ausbeute zum gewünschten Produkt (vgl. Beispiele 4 und 5).

b) 254 g K-3-Sulfopropylphenylether (1 mol) wurden wie unter a angegeben in die freie Säure übergeführt und der thermischen Spaltung unterworfen bei 140-180° C und einem Druck von 7 mb. Zwischen 90 und 120° C destillierten hierbei 0,82 mol Phenol und zwischen 160 und 167° C Dampftemperatur 0,86 mol reines 1,3-Propansulton ab.

*Beispiel 2:*

*3-Methyl-1,3-Propansulton*

a) 282 g (1 mol) K-3-(3-Methylphenoxy)-1-methylpropansulfonat wurden in 0,8 l Aceton suspendiert und mit 100 ml konzentrierter Salzsäure 30 min bei Raumtemperatur gerührt. Das ausgefallene Kaliumchlorid (98% der Theorie) wurde abgesaugt und das Filtrat wurde bei Normaldruck eingedampft. Der Rückstand wurde auf 160-170° C bei einem Druck von 7 mb erhitzt. Dabei destillierte zuerst m-Kresol bei 114-127° C/7 mb (Ausbeute: 84 g) und dann bei 154-161° C/7 mb 3-Methyl-1,3-propansulton ab. Ausbeute: 125,6 g = 92% der Theorie.

b) Durch Umsetzung von 1 mol des K-Sulfonats mit Salzsäure wie unter a beschrieben und thermische Spaltung der Ethersäure bei 160-170° C unter Abdestillation des gebildeten m-Kresols (= 0,75 mol). Im Rückstand verblieb ein Gemisch von

3-m-Kresyloxy-1-methylpropansulfonsäure (24%) und 76% 3-Methyl-1,3-Propansulton, das ohne weiteres als Sulfoalkylierungsmittel verwendet werden konnte.

*Beispiel 3:*

*1,4-Butansulton*

Analog Beispiel 2a aus 282 g Kalium-4-(3-methylphenoxy)butansulfonat.

Ausbeute: 127 g (= 93% der Theorie).

Kp.: 160° C/7 mb.

*Beispiel 4:* (erfindungsgemäss)

Anhydro-2-methyl-3-(3-sulfopropyl)-5-phenylbenzoxazoliumhydroxid; Fp. 289-290° C. 0,85 mol Phenoxypropansulfonsäure wurden durch Erhitzen wie in Beispiel 1a beschrieben unter Abdestillation von Phenol zu Propansulton cyclisiert. Nach Zugabe von 0,85 mol 2-Methyl-5-phenylbenzoxazol wurde 1,5 h auf 175° C erhitzt unter Rühren. Anschliessend wurde mit Ethanol aufgearbeitet.

Ausbeute: 230,7 g = 82% der Theorie.

*Beispiel 5:* (Vergleich)

Anhydro-2-methyl-3-(3-sulfopropyl)-5-phenylbenzoxazoliumhydroxid; Fp. 286-289° C.

Analog Beispiel 4 wurden 0,85 mol 3-Methoxypropansulfonsäure – hergestellt nach den Angaben von Helberger *et al.*, [„Liebigs Ann. Chem.", *586* (1954), 149, 154] – bei 160-165° C cyclisiert unter Abdestillation von Methanol und anschliessend bei 175° C mit 2-Methyl-5-phenylbenzoxazol umgesetzt.

Ausbeute: 140,6 g = 50% der Theorie.

**Patentansprüche**

1. Verfahren zur Herstellung von Alkansultonen der allgemeinen Formel (I):

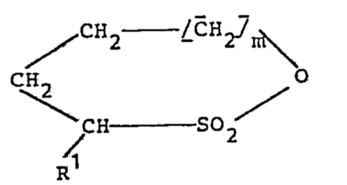

(I)

worin bedeuten

$R^1$ Wasserstoff oder Methyl, und

m 0 oder 1,

gekennzeichnet durch thermische Spaltung von Sulfoalkylarylethern der allgemeinen Formel (II) bei einer Temperatur zwischen 80 und 250° C:

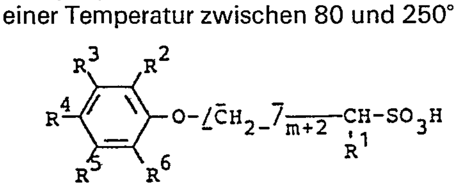

(II)

worin bedeuten

$R^1$ Wasserstoff oder Methyl,

m 0 oder 1, und

$R^2$, $R^3$, $R^4$, $R^5$ und $R^6$ Wasserstoff oder gleiche oder verschiedene Substituenten, und zwar Methyl, Ethyl, Halogen oder Alkoxy, oder

$R^3$ bedeutet zusammen mit $R^2$ oder zusammen mit $R^4$ einen Rest zur Vervollständigung eines ankondensierten 5- oder 6gliedrigen Ringes.

2. Verfahren nach Anspruch 1, dadurch gekennzeichnet, dass die thermische Spaltung zwischen 140 und 170° C durchgeführt wird.

3. Verfahren nach Anspruch 1, dadurch gekennzeichnet, dass das bei der thermischen Spaltung gebildete Phenol durch Destillation unter vermindertem Druck vollständig oder teilweise aus dem Reaktionsgefäss entfernt wird.

4. Verfahren nach Anspruch 3, dadurch gekennzeichnet, dass das Alkansulton in dem Reaktionsgefäss mit sulfoalkylierbaren Verbindungen umgesetzt wird.

5. Verfahren nach Anspruch 3, dadurch gekennzeichnet, dass das Alkansulton durch Destillation unter vermindertem Druck in eine Destillationsvorlage überführt und darin mit sulfoalkylierbaren Verbindungen umgesetzt wird.

**Claims**

1. Process for the production of alkane sultones of the general Formula (I):

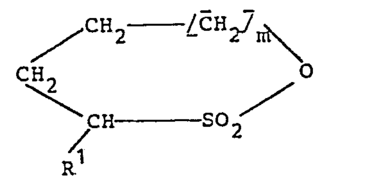

(I)

wherein:

$R^1$ denotes hydrogen or methyl, and

m denotes 0 or 1,

characterised by thermal decomposition of sulphoalkyl-aryl ethers of the general Formula (II) at a temperature between 80 and 250° C:

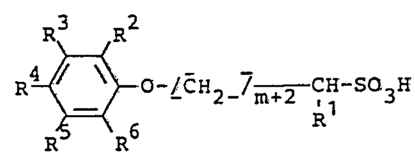

(II)

wherein:

$R^1$ denotes hydrogen or methyl,

m denotes 0 or 1, and

$R^2$, $R^3$, $R^4$, $R^5$ and $R^6$ denote hydrogen or indentical or different substituents, namely methyl, ethyl, halogen or alkoxy, or

$R^3$ together with $R^2$ or together with $R^4$ denotes a radical to complete a fused-on 5- or 6-membered ring.

2. Process according to Claim 1, characterised in that the thermal decomposition is carried out at between 140 and 170° C.

3. Process according to Claim 1, characterised in that the phenol formed during the thermal decomposition is completely or partly removed from the reaction vessel by distillation under reduced pressure.

4. Process according to Claim 3, characterised in that the alkane sultone is reacted with sulphoalkylatable compounds in the reaction vessel.

5. Process according to Claim 3, characterised in that the alkane sultone is transferred to a distillation receiver by distillation under reduced pressure and reacted therein with sulphoalkylatable compounds.

## Revendications

1. Procédé pour la fabrication d'alcanesultones de formule générale (I):

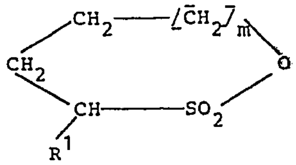

(I)

dans laquelle:

$R^1$ représente l'hydrogène ou un groupe méthyle, et

m est égal à 0 ou à 1,

caractérisé par la dissociation thermique d'éthers sulfoalkylearyliques de formule générale (II) à une température de 80 à 250° C:

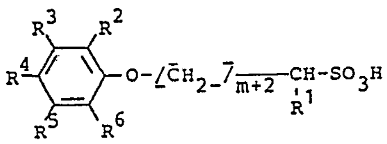

(II)

dans laquelle:

$R^1$ représente l'hydrogène ou un groupe méthyle,

m est égal à 0 ou à 1, et

$R^2$, $R^3$, $R^4$, $R^5$ et $R^6$ sont l'hydrogène ou des substituants identiques ou différents, à savoir méthyle, éthyle, halogène ou alcoxy, ou bien

$R^3$ pris ensemble avec $R^2$ ou avec $R^4$ représente un reste pour compléter un noyau condensé à 5 ou 6 chaînons.

2. Procédé selon la revendication 1, caractérisé en ce que la dissociation thermique est effectuée entre 140 et 170° C.

3. Procédé selon la revendication 1, caractérisé en ce que le phénol formé dans la dissociation thermique est éliminé totalement ou partiellement du récipient de réaction par distillation sous pression réduite.

4. Procédé selon la revendication 3, caractérisé en ce que l'alcanesultone est mise à réagir dans le récipient de réaction avec des composés sulfoalkylables.

5. Procédé selon la revendication 3, caractérisé en ce que l'alcanesultone est envoyée par distillation sous pression réduite dans un récipient récepteur de distillations et mise à réagir dans celui-ci avec des composés sulfoalkylables.